# EUROPÄISCHE PATENTSCHRIFT

(11) **EP 0 302 948 B1**
(45) Veröffentlichungstag und Bekanntmachung des Hinweises auf die Patenterteilung: **01.12.1993**
(21) Anmeldenummer: 87110541.7
(22) Anmeldetag: 21.07.1987
(51) Int. Cl.: C07H 15/256, A23L 1/22

(54) **Verfahren zur Gewinnung von Steviosiden aus pflanzlichem Rohmaterial**
Process for obtaining steviosides from plants
Procédé d'obtention de stéviosides à partir de plantes

(43) Veröffentlichungstag der Anmeldung: 15.02.1989
(73) Patentinhaber: Giovanetto, Roger H., T2E6P7 Calgary Alberta (CA)
(72) Erfinder: Giovanetto, Roger H., T2E6P7 Calgary Alberta (CA)
(74) Vertreter: Matschkur, Götz, Lindner Patent- und Rechtsanwälte

(56) Entgegenhaltungen:
- CHEMICAL ABSTRACTS, Band 83, Nr. 1, 7. Juli 1975, Seite 473, Ref. Nr. 5377b, Columbus, Ohio, US; & JP-A-75 24 300 (TAMA BIOCHEMICAL RESEARCH CO. LTD) 15-03-1975
- CHEMICAL ABSTRACTS, Band 97, Nr. 3, 19. Juli 1982, Seite 459, Ref.Nr. 20962w, Columbus, Ohio, US; & JP-A-82 46 998 (FUJI FOODS K.K.) 17-03-1982
- CHEMICAL ABSTRACTS, Band 101, Nr. 20, 12. November 1984, Seite 377, Ref.Nr. 177332q, Columbus, Ohio, US; J. HUANG et al.: "Extraction and isolation of stevioside from leaves of Stevia rebaudiana Bertoni indigenous to China", & ZHONGCAOYAO, 1984, 15(8), 379
- CHEMICAL ABSTRACTS, Band 103, Nr. 11, 16. September 1985, Seite 512, Ref.Nr. 86668d, Columbus, Ohio, US; R. ZHOU et al.: "Ion exchange methods in extraction and purification of steviosides from Stevia rebaudiana", & ZHONGGUO TIAOUEIPIN, 1984, (12), 12-13
- CHEMICAL ABSTRACTS, Band 103, Nr. 21, 25. November 1985, Seite 578, Ref.Nr. 177127t, Columbus, Ohio, US; R.R. DE CERNADAS et al.: "A method for the isolation of stevioside from leaves of Stevia rebaudiana Bert.", & REV. AGROQUIM. TECNOL. ALIMENT., 1985, 25(2), 268-72
- CHEMICAL ABSTRACTS, Band 108, Nr. 19, 9. Mai 1988, Seite 550, Ref.Nr. 166353r, Columbus, Ohio, US; & BR-A-87 00 543 (H. ARASHI) 16-06-1987
- CHEMICAL ABSTRACTS, Band 86, Nr. 1, 3. Januar 1977, Seite 504, Ref.Nr. 5777u, Columbus, Ohio, US; & JP-A-76 91 300 (NIKKEN CHEMICALS CO. LTD) 10-08-1976
- CHEMICAL ABSTRACTS, Band 90, Nr. 23, 4. Juni 1979, Seite 305, Ref.Nr. 182809j, Columbus, Ohio, US; & JP-A-79 20 000 (JAPAN ORGANO CO. LTD) 15-02-1979
- CHEMICAL ABSTRACTS, Band 93, Nr. 5, 4. August 1980, Seite 742, Ref.Nr. 44371w, Columbus, Ohio, US; & JP-A-80 39 731 (INSTITUTE FOR PRODUCTION AND DEVELOPMENT SCIENCE) 19-03-1980

## Beschreibung

Die Erfindung bezieht sich auf ein Verfahren zur Gewinnung von Steviosiden aus getrocknetem pflanzlichen Material von Stevia rebaudiana Bertoni unter Extraktion und Reinigung.

Stevioside werden als brauchbare künstliche Süßstoffe verwendet und kalorienarmen Nahrungsmitteln zugesetzt oder als Ersatz für natürlichen Zucker gebraucht. Künstliche Süßstoffe wurden entwickelt zum Gebrauch für Diabetiker und zur Verminderung des Kaloriengehalts von Nahrungsmittelzubereitungen, insbesondere für eine kalorienarme Diät. Diese Süßstoffe sind vielfach süßer als der natürliche Zucker und können, um das gleiche Maß an Süßwirkung zu erzielen, in geringen Mengen verwendet werden. Zahlreiche Süßstoffe sind synthetischer Natur, wie z.B. Saccharin, Cyclamate und Aspartame. Einige hiervon sind verboten oder in ihrem Gebrauch eingeschränkt, weil pharmakologische Untersuchungen ergeben haben, daß sie Krebs erzeugen können. Die Stevioside dagegen sind voll verwendbar und haben in klinischen Tests keine nachteiligen Wirkungen gezeigt.

Gebräuchliche Verfahren zur Extraktion und Reinigung von Steviosiden sind fast ausschließlich verbunden mit der Verwendung organischer Lösungsmittel, wie Methanol, Äthanol oder Äther, und viele erfordern zunächst die Absorption der Stevioside an ein Harz unter nachfolgender Elution mit einem organischen Lösungsmittel. Die aus diesem Verfahren konzentrierten eingedickten Lösungen werden üblicherweise mit Methanol oder Äthanol behandelt, um die schließliche Kristallisation des Endgemisches zu erreichen. Andere Verfahren bedienen sich Eisen- oder Aluminiumsalze, um Verunreinigungen zu entfernen. Diese beiden Mittel erfordern eine weitere Behandlung mit Natriumhydroxid zur Entfernung von Resten der Eisen- oder Aluminiumsalze, man vergleiche hierzu beispielsweise Chemical Abstracts, Band 101, Nr. 20, 12. November 1984, Seite 377, Ref. Nr. 17 73 32q, Chemical Abstracts, Band 103, Nr. 11, 16. September 1985, Seite 512, Ref. Nr. 86 668d und insbesondere Chemical Abstracts, Band 83, Nr. 1, 7. Juli 1975, Seite 473, Ref. Nr. 5377b.

Ausgehend von der in der vorstehend beschriebenen Druckschrift Chemical Abstracts, Band 83 genannten Entgegenhaltung liegt der vorliegenden Erfindung die Aufgabe zugrunde, ein Verfahren bereitzustellen mit besseren Ergebnissen (höhere Reinheit und Geschmacksverbesserung des Steviosids), ohne daß dies auf Kosten der Ausbeute geschieht.

Zur Lösung dieser Aufgabe ist bei dem bekannten Verfahren der eingangs genannten Art gemäß der vorliegenden Erfindung vorgesehen, daß das Filtrat zunächst mit einem stark sauren Ionenaustauschharz und anschließend mit einem schwach basischen Ionenaustauschharz behandelt wird und diese Behandlungsreihenfolge mehrfach, vorzugsweise dreifach, wiederholt wird.

Bei dem erfindungsgemäßen Verfahren besteht das einzige chemische Additiv aus einem Zusatz von Calciumhydroxid zum Ausgangsmaterial, wodurch die Masse der unerwünschten Verunreinigungen und Farbstoffe entfernt wird. Alle weiteren Verfahrensschritte werden mit Wasser als Lösungsmittel ausgeführt und das Erzeugnis wird durch Verdampfen des Wassers und Trocknung gewonnen, also ein Steviosid von hoher Reinheit, gutem Geschmack und einwandfreier Färbung, sowie mit einer hohen Gesamtausbeute gegenüber dem Rohmaterial. Eine wesentliche Besonderheit des Verfahrens besteht dabei in der wiederholten Behandlung, da auf diese Art und Weise ohne großen Aufwand und vor allem unter Einsparung unerwünschter chemischer Zusatzstoffe sowohl der Geschmack als auch die Farbe fortlaufend verbessert werden können, indem anhand der Kation- und Anionbehandlungen die Abfallstoffe systematisch abgesondert werden.

Als saure Ionenaustauscher kommen die unter dem Handelsnamen Domex 50W, Rohm und Haas IRA 120 oder wirkungsgleiche Harze in Betracht. Die basischen Ionenaustauscherharze können beispielsweise die unter dem Handelsnamen Domex WGR, Domex MWA-1, Rohm und Haas IR4B oder Rohm und Haas IRA93 bekannten oder wirkungsgleiche Harze sein. Die Lösung wird durch ein feines Filtersystem geführt und eine Probe zur Ermittlung der Reinheit und Qualität verdampft. Entsprechend der gewünschten Reinheit und Qualität des Endprodukts kann dieses Verfahren einschließlich der Anwendung der stark sauren Ionenaustauscherharze und der schwachen basischen Ionenaustauscherharze mehrfach, im allgemeinen bis dreifach, wiederholt werden. Die Lösung wird dann konzentriert und gefiltert. Das Filtrat wird getrocknet, und es wird ein weißes Pulver gewonnen, welches etwa 75 % Steviosid-Verbindungen enthält.

Es liegt im Rahmen der Erfindung, daß der Extrakt zur Bildung des Niederschlags mit Calciumoxid, Calciumcarbonat oder anderen basischen Calciumsalzen behandelt wird. Eine andere Alternative hierzu bildet die Behandlung des Extraktes zur Bildung des Niederschlags mit basischen Salzen des Magnesiums oder Aluminiums. Vorteilhafterweise werden die basischen Salze während der Extraktion und unter Rühren dem Wasser zugesetzt.

Schließlich sieht die Erfindung vor, daß nach Behandlung mit dem stark sauren Ionenaustauscherharz und einem schwachen basischen Ionenaustauscherharz das gewonnene Filtrat mit einem stark sauren Ionenaustauscherharz sowie anschließend einem starken basischen ionenaustauscherharz behandelt und die gewonnene Lösung konzentriert, gefiltert und getrocknet wird.

Das erfindungsgemäße Verfahren wird anhand der folgenden Beispiele im einzelnen erläutert:

### Beispiel 1:

Das Rohmaterial, welches die Stevioside enthält, wird durch Wasserextraktion aus einem Kilogramm Pflanzenmaterial bei Temperaturen in der Größenordnung von Raumtemperatur bis 65°C gewonnen. Ohne zusätzliche Konzentration wird dieser Extrakt mit Calciumhydroxid behandelt, um eine Anzahl unerwünschter Pflanzenbestandteile zu entfernen, und das Ganze wird in einem feinen Filtersystem filtriert. Das Ergebnis ist eine klare Lösung, die 279 Gramm Roh-Stevioside enthält. Diese klare Lösung wird dann mit einem stark sauren Ionenaustauscherharz, wie z.B. Dowes 5OW, behandelt und das gewonnene Eluat dann mit einem schwachen basischen Ionenaustauscherharz, wie z.B. Domex WGR od.dgl.behandelt. Das aus dieser Behandlung hervorgehende Eluat wird gefiltert durch ein feines Filtermedium, bevor der nächste Verfahrensschritt folgt. Die Aufeinanderfolge der Behandlungen mit stark sauren Harz und schwachem basischen Harz wird so lange wiederholt, bis ein befriedigendes Produkt erlangt wird. Dies kann sich von einer bis zur fünfmaligen Behandlung erstrecken. Nach derElution und Filtration am Ende der letzten Ionenaustauscherbehandlung wird das gewonnene Eluat konzentriert und in einem feinen Filtersystem gefiltert. Das Filtrat wird getrocknet und ergibt ein gereinigtes Produkt mit 107 Gramm von etwa 75 % reinen Steviosiden.

### Beispiel 2

Dieses Verfahren entspricht im wesentlichen dem Beispiel 1, jedoch wird das Eluat nach dreifach aufeinanderfolgender Behandlung mit einem stark sauren Harz und einem schwachen basischen Harz und Filtration konzentriert. Das Konzentrat wird dann ein weiteres Mal mit einem entsprechend starken sauren Harz und anschließend mit einem stark basischen Harz, wie z.B. Dowex 2-X oder Rohm und Haas IRA410 od.dgl. behandelt. Das Ergebnis dieser Behandlung wird dann konzentriert und das Konzentrat in einem Feinfiltersystem gefiltert. Das Endprodukt gewinnt man durch Trocknen, es ergibt 102 Gramm eines gereinigten Erzeugnisses, welches etwa 75 % reine Stevioside enthält, jedoch weniger bitter bei der Geschmacksprüfung ist.

## Patentansprüche

1. Verfahren zur Gewinnung von Steviosiden aus getrocknetem pflanzlichen Material von Stevia rebaudiana Bertoni wobei durch Behandlung in Wasser bei einer Temperatur von Raumtemperatur bis ca. 65°C und unter Rühren sowie anschließender Filtration oder Zentrifugieren ein Extrakt gewonnen und dieser mit Calciumhydroxid behandelt wird, worauf mittels Filtration oder Zentrifugieren ein Niederschlag gewonnen und das Filtrat mit Ionenaustauscherharzen behandelt und die gewonnene Lösung konzentriert, gefiltert und getrocknet wird, dadurch gekennzeichnet, daß das Filtrat zunächst mit einem stark sauren Ionenaustauscherharz und anschließend mit einem schwach basischen Ionenaustauschharz behandelt wird und diese Behandlungsreihenfolge mehrfach, vorzugsweise dreifach, wiederholt wird.

2. Verfahren nach Anspruch 1, dadurch gekennzeichnet, daß der Extrakt zur Bildung des Niederschlags mit Calciumoxid, Calciumcarbonat oder anderen basischen Salzen behandelt wird.

3. Verfahren nach Anspruch 1, dadurch gekennzeichnet, daß der Extrakt zur Bildung des Niederschlages mit basischen Salzen des Magnesiums oder Aluminiums behandelt wird.

4. Verfahren nach einem der Ansprüche 1 bis 3, dadurch gekennzeichnet, daß die basischen Salze während der Extraktion und unter Rühren dem Wasser zugesetzt werden.

5. Verfahren nach einem der Ansprüche 1 bis 4, dadurch gekennzeichnet, daß das nach Behandlung mit einem stark sauren Ionenaustauscherharz und mit einem schwachen basischen Ionenaustauscherharz gewonnene Filtrat mit einem stark sauren Ionenaustauscherharz sowie anschließend mit einem stark basischen Ionenaustauscherharz behandelt, daß das gewonnene Zwischenprodukt konzentriert, gefiltert und getrocknet wird.

6. Verfahren nach einem der Ansprüche 1 bis 5, dadurch gekennzeichnet, daß das aus dem Rohextrakt gewonnene Filtrat mit den Ionenaustauscherharzen intermittierend behandelt wird.

## Claims

1. Process for obtaining steviosides from dried plant material from *Stevia rebaudiana Bertoni,* wherein by treatment in water at a temperature from room temperature up to approx. 65°C and subject to stirring and subsequent filtration or centrifuging, an extract is obtained which is then treated with calcium hydroxide, whereupon by means of filtration or centrifuging a precipitate is obtained, and the filtrate is treated with ion-exchange resins and the solution obtained is concentrated, filtered and dried, characterised in that the filtrate is first treated with a strongly acidic ion-exchange resin and then with a weakly basic ion exchange resin, and this treatment sequence is repeated more than once, preferably three times.

2. Process according to claim 1, characterised in that the extract is treated with calcium oxide, calcium carbonate or other basic salts in order to form the precipitate.

3. Process according to claim 1, characterised in that the extract for the formation of the precipitate is treated with basic salts of magnesium or aluminium.

4. Process according to one of claims 1 to 3, characterised in that the basic salts are added to the water during extraction and whilst being stirred.

5. Process according to one of claims 1 to 4, characterised in that the filtrate obtained after treatment with a strongly acidic ion exchange resin and with a weakly basic ion exchange resin is treated with a strongly acidic ion exchange resin and then with a weakly basic ion exchange resin, and in that the intermediate product obtained is concentrated, filtered and dried.

6. Process according to one of claims 1 to 5, characterised in that the filtrate obtained from the raw extract is intermittently treated with the ion exchange resins.

## Revendications

1. Procédé d'obtention de stéviosides à partir d'une matière végétale séchée de stevia rebaudiana Bertoni selon lequel on obtient par traitement dans l'eau à une température allant de la température ambiante à 65°C environ et sous agitation ainsi qu'avec une filtration subséquente ou une centrifugation subséquente un extrait que l'on traite par l'hydroxyde de calcium, puis l'on obtient par une filtration ou une centrifugation un précipité et l'on traite le filtrat par des résines échangeuses d'ions et l'on concentre, filtre et sèche la solution obtenue, procédé caractérisé en ce qu'on traite le filtrat tout d'abord avec une résine échangeuse d'ions fortement acide, puis avec une résine échangeuse d'ions faiblement basique et l'on répète plusieurs fois cette suite de traitements, avantageusement trois fois.

2. Procédé selon la revendication 1, caractérisé en ce que, pour former le précipité, on traite l'extrait avec de l'oxyde de calcium, du carbonate de calcium ou d'autres sels basiques.

3. Procédé selon la revendiccation 1, caractérisé en ce qu'on traite l'extrait, pour former le précipité, avec des sels basiques du magnésium ou de l'aluminium.

4. Procédé selon l'une des revendications 1 à 3, caractérisé en ce qu'on ajoute à l'eau les sels basiques pendant l'extraction et en opérant sous agitation.

5. Procédé selon l'une des revendications 1 à 4, caractérisé en ce qu'après le traitement par une résine échangeuse d'ions fortement acide et avec une résine échangeuse d'ions faiblement basique, on soumet le filtrat ainsi obtenu à un traitement par une résine échangeuse d'ions fortement acide ainsi que, ensuite, par une résine échangeuse d'ions fortement basique, on concentre, filtre et sèche ensuite le produit intermédiaire ainsi obtenu.

6. Procédé selon l'une des revendications 1 à 5, caractérisé en ce qu'on traite de façon intermittente par des résines échangeuses d'ions le filtrat obtenu à partir de l'extrait brut.
